# EUROPÄISCHE PATENTSCHRIFT

(11) **EP 0 344 096 B1**
(45) Veröffentlichungstag und Bekanntmachung des Hinweises auf die Patenterteilung: **05.01.1994**
(21) Anmeldenummer: 89730124.8
(22) Anmeldetag: 19.05.1989
(51) Int. Cl.: C07C 33/30, C07C 59/48, C07C 69/708, C07C 69/73

(54) **Neue Leukotrien-B4-Derivate, Verfahren zu ihrer Herstellung und ihre Verwendung als Arzneimittel**
Leukotriene B4 derivatives, their preparation and use as medicines
Dérivés de la leukotriène-B4, leur procédé et application thérapeutique

(30) Priorität: 19.05.1988 DE 3817385
(43) Veröffentlichungstag der Anmeldung: 29.11.1989
(73) Patentinhaber: SCHERING AKTIENGESELLSCHAFT, 13342 Berlin (DE)
(72) Erfinder: Skuballa, Werner, D-1000 Berlin 28 (DE); Buchmann, Bernd, D-1000 Berlin 21 (DE); Fröhlich, Wolfgang, D-1000 Berlin 31 (DE); Ekerdt, Roland, D-1000 Berlin 28 (DE)

(56) Entgegenhaltungen:
- EP-A- 0 296 580
- EP-A- 0 319 900

## Beschreibung

Die Erfindung betrifft neue Leukotrien-B₄-Derivate, Verfahren zu ihrer Herstellung sowie ihre Verwendung als Arzneimittel.

Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird. 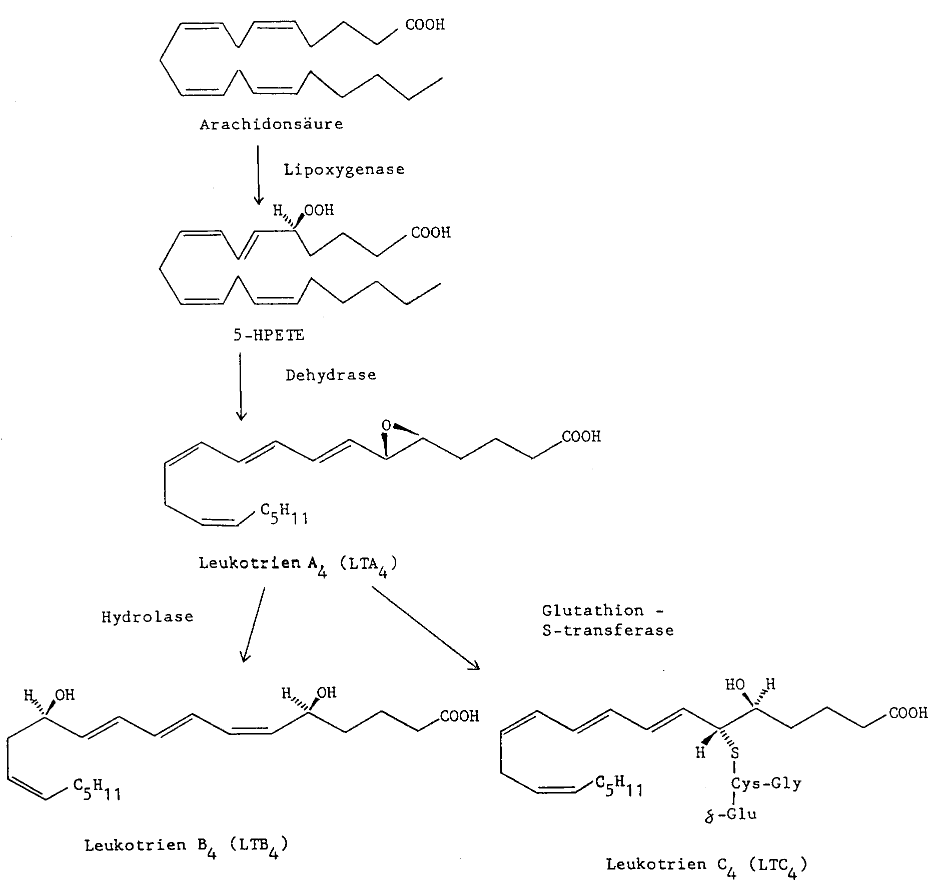
Die Nomenklatur der Leukotriene kann folgenden Arbeiten entnommen werden: a) B. Samuelsson et al., Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987).

Vom LTB₄ weiß man, daß es die Adhäsion von Leukozyten an die Blutgefäßwand verursacht. LTB₄ hat chemotaktische Wirkungen, d.h. es zieht Leukozyten in einem Gradienten steigender Konzentration an. Außerdem verändert es aufgrund seiner chemotaktischen Aktivität indirekt die vasculäre Permeabilität, wobei ein Synergismus mit Prostaglandin E₂ beobachtet wurde. LTB₄ spielt offensichtlich bei entzündlichen, allergischen und immunologischen Prozessen eine entscheidende Rolle. Antagonisten gegen LTB₄ selbst oder Inhibitoren jener Enzyme, die an der Synthese des LTB₄ beteiligt sind, sollten als spezifische Heilmittel, besonders gegen Krankheiten, die mit Entzündungen und allergischen Reaktionen einhergehen, wirksam sein. Neben therapeutischen Möglichkeiten, die sich aus einer Antagonisierung des LTB₄ mit LTB₄-Analoga ableiten lassen, konnte kürzlich auch die Nützlichkeit und potentielle Anwendung von Leukotrien B₄-Agonisten zur Behandlung von Pilzerkrankungen der Haut gezeigt werden (H. Kayama, Prostaglandins 34, 797 (1988)).

Insbesondere sind die Leukotriene an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Spiegel sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienspiegel wurden beispielsweise bei Psoriasis und atopischer Dermatitis gemessen.

Es wurde nun gefunden, daß der Einbau der chemisch und metabolisch labilen cis-Δ^{6,7}-Doppelbindung des LTB₄ in einen 1,2-substituierten Phenylring zu einer Stabilisierung des LTB₄ führt, wobei insbesondere durch weitere Derivatisierung der funktionellen Gruppen, auch LTB₄-Derivate erhalten werden, die die Wirkung des natürlichen LTB₄ stark antagonisieren. Das heißt, die Erfindung umfaßt LTB₄-Analoga, die je nach Strukturcharakteristik und Gewebeart als Antagonisten, Agonisten und Partialantagonisten wirken können.

Die Erfindung betrifft Leukotrien-B₄-Derivate der Formel I, 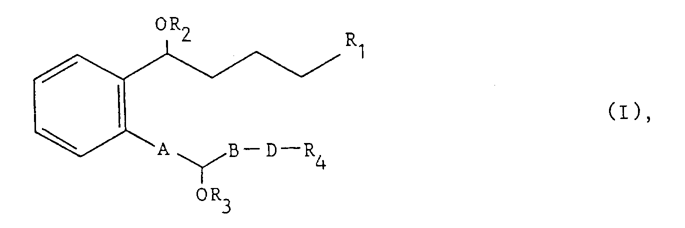
worin
- R₁: den Rest CH₂OH, den Rest COOR₅ mit R₅ in der Bedeutung eines Wasserstoffatoms, eines Alkylrestes mit 1-10 C-Atomen, eines Cycloalkylrestes mit 3-10 C-Atomen, eines gegebenenfalls durch 1-3 Chlor, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrestes mit 6-10 C-Atomen, eines -CH₂-CO-Aryl-Restes mit 6-10 C-Atomen für Aryl oder eines 5-6-gliedrigen heterocyclischen Restes mit wenigstens 1 Heteroatom oder den Rest CONHR₆ mit R₆ in der Bedeutung eines Alkanoyl- oder Alkansulfonylrestes mit 1-10 C-Atomen oder des Restes R₅,
- A: eine trans, trans-CH=CH-CH=CH- oder Tetramethylengruppe,
- B: eine geradkettige oder verzweigtkettige gesättigte oder ungesättigte Alkylengruppe mit bis zu 10 C-Atomen, die gegebenenfalls durch Fluor substituiert sein kann, oder die Gruppe 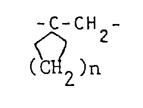 mit n = 1, 2 oder 3,
- D: eine Direktverbindung, Sauerstoff, Schwefel, eine -C≡C-Gruppe oder eine -CH=CR₇-Gruppe mit R₇ als Wasserstoff, C₁-C₅-Alkyl, Chlor oder Brom,
- B und D: gemeinsam eine direkte Bindung,
- R₂ und R₃: gleich oder verschieden sein können und Wasserstoffatom oder einen Säurerest einer organischen Säure mit 1-15 C-Atomen und
- R₄: ein Wasserstoffatom, einen Alkylrest mit 1-10 C-Atomen, einen durch Chlor oder Brom substituierten Alkylrest mit 1-10 C-Atomen, einen Cycloalkylrest mit 3-10 C-Atomen, einen gegebenenfalls durch 1-2 Chlor-, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrest mit 6-10 C-Atomen oder einen 5-6-gliedrigen heterocyclischen Rest mit wenigstens 1 Heteroatom bedeuten
und, falls R₅ die Bedeutung eines Wasserstoffatom hat, deren Salze mit physiologisch verträglichen Basen und deren Cyclodextrinclathrate,
mit der Maßgabe, daß R₂ und R₃ nicht jeweils Wasserstoff bedeuten, falls A eine trans,trans-CH=CH-CH=CH- und R₁den Rest -COOR₅ oder -CONHR₆ darstellen.
Verbindungen der Formel I mit R₂ und R₃ in der Bedeutung von jeweils Wasserstoff und A in der Bedeutung von -CH=CH-CH=CH sowie R₁in der Bedeutung des Restes -COOR₅ oder -CONHR₆ sind in der EP-A 0319 900 vorbeschreiben, die aber zum Prioritätsdatum der vorliegenden Anmeldung noch nicht publiziert war.

Als Alkylgruppen R₅ kommen gerad- oder verzweigtkettige Alkylgruppen mit 1-10 C-Atomen in Betracht, wie beispielsweise Methyl, Ethyl, Propyl, Butyl, Isobutyl, tert.-Butyl, Pentyl, Neopentyl, Hexyl, Heptyl, Decyl. Die Alkylgruppen R₅ können gegebenenfalls ein- bis mehrfach substituiert sein durch Halogenatome, Alkoxygruppen, gegebenenfalls substituierte Aryl- bzw. Aroylgruppen, Dialkylamino und Trialkylammonium mit jeweils 1-4 C-Atomen im alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R₅ sind solche mit 1-4 C-Atomen zu nennen.

Als Aryl-bzw. Aroylgruppen kommen die nachstehend genannten Aryle in Betracht.

Als Arylgruppen R₅ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome (F, Cl, Br) , eine Phenylgruppe, 1-3 Alkylgruppen mit jeweils 1-4 C-Atomen, eine Chlormethyl-, Fluormethyl-, Trifluormethyl, - Carboxyl-, Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy.

Die Cycloalkylgruppe R₅ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl.

Als heterocyclische Gruppen R₅ kommen 5- und 6-gliedrige aromatische Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R₆ kommen physiologisch verträgliche Säurereste in Frage. Bevorzugte Säuren sind organische Carbonsäuren und Sulfonsäuren mit 1-15 Kohlenstoffatomen. die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seinen Alkyl-, Hydroxy-, Alkoxy- , Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure. Phenoxyesigsäure, Methoxyessigsäure, Ethoxyessigsäure, Mono-Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy , - Alkoxy- oder CarboxyGruppen substituierte Benzoesäuren, Nikotinsäure, Isoniktonsäure, Furan-2-carbonsäure, Cyclopentylpropionsäure. Als besonders bevorzugte Acylreste und Alkansulfonylreste werden solche mit bis zu 10 Kohlenstoffatomen betrachtet. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Als Alkylgruppen R₄ kommen gerad- und verzweigtkettige, gesättigte und ungesättigte Alkylreste, vorzugsweise gesättigte, mit 1-10, insbesondere 1-6 C-Atomen, in Frage, die gegebenenfalls durch gegebenenfalls substituiertes Aryl substituiert sein können. Beispielsweise genannt seien Methyl-, Ethyl-, Propyl-, Butyl- , Isobutyl-, tert.,-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen R₄ Halogen-substituiert, kommen als Halogene Fluor, Chlor und Brom in Frage.

Die Cycloalkylgruppe R₄ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seinen Cyclopropyl, Cyclobutyl, Cyclopentyl, Cyclohexyl, Methyl-cyclohexyl.

Als substituierte bzw. unsubstituierte Arylgruppen R₄ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen eine Chlormethyl-, Fluormethyl-, Trifluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische Gruppen R₄ kommen 5- und 6-gliedrige aromatische Heterocyclen in Frage, die wenigstens 1 Heteroatom, vorzugsweise Stickstoff, Sauerstoff oder Schwefel enthalten. Beispielsweise seien genannt 2-Furyl, 2--Thienyl, 2-Pyridyl, 3-Pyridyl, 4-Pyridyl, Oxazolyl, Thiazolyl, Pyrimidinyl, Pyrldazinyl, Pyrazinyl, 3-Furyl, 3-Thienyl, u.a.

Als Alkylengruppe B kommen geradkettige oder verzweigtkettige, gesättigte und ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1.1-Difluorethylen, 1-Fluorethylen, 1-Methyltetramethylen, 1-Methyl-tri-methylen, 1-Methylen-ethylen, 1-Methylen-tetramethylen.

Als Säurereste R₂ und R₃ kommen physiologisch verträgliche Säurereste in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien Alkyl-, Hydroxy-, Alkoxy, Oxo- oder Aminogruppen oder Halogenatome erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyesigsäure, Methoxyessigsäure, Ethoxyessigsäure, Mono-, Di- und Trichloressigsäure, Amlnoessigsäure, Diethylaminoessigsäure, Piperidinoessigsäure, Morpholinoessigsaure, Milchsäure, Bernsteinsäure, Adipinsäure, Benzoesäure, mit Halogen-, Trifluormethyl-, Hydroxy,- Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Säurereste R₂ und R₃ werden Acylreste mit bis zu 10 Kohlenstoffatomen betrachtet.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Zur Herstellung der erfindungsgemäßen Clathrate werden die Verbindungen der allgemeinen Formel I in einem pharmakologisch unbedenklichen Lösungsmittel, z.B. Alkohol, vorzugsweise Ethanol, einem Keton, z.B. Aceton oder einem Ether, z.B. Diethylether, aufgelöst und mit wäßrigen Lösungen von α-, β- oder γ-Cyclodextrin, vorzugsweise β-Cyclodextrin, bei 20-80°C vermischt, oder es werden die Säuren in Form der wäßrigen Lösungen ihrer Salze, z.B. der Natrium- oder Kaliumsalze mit dem Cyclodextrin versetzt und nach Lösung mit der äquivalenten Menge einer Säure, z.B. Salzsäure oder Schwefelsäure, versetzt.

Außerdem betrifft die Erfindung ein Verfahren zur Herstellung der erfindungsgemäßen Leukotrien-B₄-Derivate der Formel I, das dadurch gekennzeichnet ist, daß man einen Benzaldehyd der Formel II, 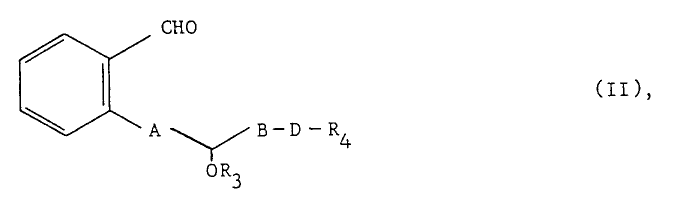
worin A, B, D, R₃ und R₄ die oben angegebenen Bedeutungen haben, gegebenenfalls nach Schutz freier Hydroxygruppen mit einer magnesiumorganischen Verbindung der Formel III,

X-Mg-CH₂-CH₂-CH₂-CH₂-O-R₈ (III),

worin X Chlor, Brom oder Jod und R₈ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R₁=COOR₅) verseift und/oder reduziert und/oder eine Carboxylgruppe (R₅=H) verestert und/oder eine freie Carboxylgruppe (R₅=H) in ein Amid (R₁=CONHR₆) überführt oder eine Carboxygruppe mit einer physiologisch verträglichen Base in ein Salz überführt.

Als Etherrester R₈ in der Verbindung der Formel III kommen die dem Fachmann geläufigen Reste in Betracht. Bevorzugt sind leicht abspaltbare Etherreste, wie beispielsweise Dlmethyl-tert.-butylsilyl, Trimethylsilyl-, Tribenzylsilyl-, Diphenyl-tert.-butylsilyl-, Tetrahydropyranyl-, Tetrahydrofuranyl- und α-Ethoxyethyl, um nur einige zu nennen.

Die Umsetzung der Verbindung der Formel II mit einer metallorganischen Verbindung der Formel III erfolgt in an sich bekannter Weise in einem inerten Lösungsmittel oder Lösungsmittelgemisch, wie zum Beispiel Diethylether, Tetrahydrofuran, Dioxan, Toluol, Dimethoxyethan, vorzugsweise Diethylether oder Tetrahydrofuran. Die Reaktion wird bei Temperaturen zwischen -100 °C und 60 °C, vorzugsweise bei -78 °C bis 0 °C durchgeführt.

Die Herstellung der für diese Umsetzung benötigten Verbindung der Formel III erfolgt durch Reaktion des entsprechenden Hydroxyhalogenids durch Veretherung mit Dihydropyran und p-Toluolsulfonsäure und anschließende Umsetzung mit Magnesium.

Die Reduktion zu den Verbindungen der Formel I mit R₁ in der Bedeutung einer -CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeteten Lösungsmittels, vorzugsweise 0 °C bis 30 °C vorgenommen.

Die Veresterung der Alkohole der Formel I (R₂ = H und/oder R₃ = H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise Na-Hydrid, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, DMSO bei Temperaturen über oder unter Raumtemperatur, zum Beispiel zwischen -80 °C bis 100 °C, vorzugsweise bei Raumtemperatur, vorgenommen werden.

Die Oxidation der l-Hydroxygruppe wird nach den dem Fachmann bekannter Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation und anschließende Jones-Oxidation. Die Oxidation mit Pyridiniumchromat wird bei Temperaturen von 0 °C bis 100 °C, vorzugsweise 20 °C bis 40 °C in einem gegen das Oxidationsmittel inerten Lösungsmittel, beispielsweise Dimethylformamid, durchgeführt.

Die Oxidation mit Jones-Reagenz wird bei Temperaturen von -40 °C bis +40 °C, vorzugsweise 0 °C bis 30 °C in Aceton als Lösungsmittel ausgeführt.

Die Oxidation mit Platin/Sauerstoff wird bei Temperaturen von 0 °C bis 60 °C, vorzugsweise 20 °C bis 40 °C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Freisetzung der funktionell abgewandelten Hydroxygruppen erfolgt nach bekannten Methoden. Beispielweise wird die Abspaltung von Hydroxyschutzgruppen, wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z.B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20 °C und 80 °C durchgeführt. Die Abspaltung der Silyletherschutzgruppen erfolgt beispielsweise mit Tetrabutylammoniumfluorid oder mit Kaliumfluorid in Gegenwart eines Kronenethers. Als Lösungsmittel sind beispielsweise geeignet Tetrahydrofuran, Diethylether, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohol kommen aliphatische Alkohole in Betracht, wie z.B. Methanol, Ethanol, Butanol usw., vorzugsweise Methanol. Als Alkalicarbonate und -hydroxyde seien Kalium- und Natriumsalze genannt. Bevorzugt sind die Kaliumsalze.

Als Erdkalicarbonate und -hydroxide sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe 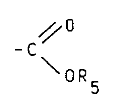
für R₁, bei welcher R₅ eine Alkylgruppe mit 1-10 C-Atomen darstellt, erfolgt nach den dem Fachmann bekannten Methoden. Die l-Carboxy-verbindungen werden beispielsweise mit Diazokohlenwasserstoffen in an sich bekannter Weise umgesetzt. Die Veresterung mit Diazokohlenwasserstoffen erfolgt z.B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Einführung der Estergruppe 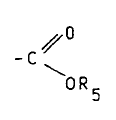
für R₁, bei welcher R₂ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base. beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C. vorzugsweise bei 10 °C, durchgeführt.

Sollen im Primärprodukt enthaltene C=C-Doppelbindungen reduziert werden, erfolgt die Hydrierung nach an sich bekannten Methoden.

Die Hydrierung des Δ^{8,10}-Diensystems wird in an sich bekannter Weise bei tiefen Temperaturen, vorzugsweise bei etwa -20 °C bis +30 °C in einer Wasserstoffatmosphäre in Gegenwart eines Edelmetallkatalysators durchgeführt. Als Katalysator ist zum Beispiel 10 % Palladium auf Kohle geeignet.

Die Leukotrien-B₄-Derivate der Formel I mit R₅ in der Bedeutung eines Wasserstoffatoms können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LT-B₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe 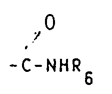
für R₁ erfolgt nach den dem Fachmann bekannten Methoden. Die Carbonsäuren der Formel I (R₅=H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak (R₆=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30 °C und +60 °C, vorzugsweise bei 0 °C bis 30 °C.

Eine weitere Möglichkeit für die Einführung der Amidgruppe 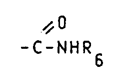
für R₁ besteht in der Umsetzung einer 1-Carbonsäure der Formel I (R₅=H), in der freie Hydroxygruppen gegebenenfalls intermediär geschützt sind, mit Verbindungen der Formel IV,

O = C = N - R₆ (IV),

worin R₆ die oben angegebene Bedeutung hat.

Die Umsetzung der Verbindung der Formel I (R₅=H) mit einem Isocyanat der Formel IV erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80 °C bis 100 °C, vorzugsweise bei 0 °C bis 30 °C, vorgenommen werden.

Enthält das Ausgangsprodukt OH-Gruppen im Leukotrien-B₄-Rest so werden diese OH-Gruppen auch zur Raktion gebracht. Werden letztlich Endprodukte gewünscht, die freie Hydroxylgruppen enthalten, geht man zweckmässigerweise von Ausgangsprodukten aus, in denen diese durch vorzugsweise leicht abspaltbare Ether- oder Acylreste intermediär geschützt sind.

Die als Ausgangsmaterial dienenden Verbindungen der Formel II können beispielsweise hergestellt werden, indem man in an sich bekannter Weise 2-(Hydroxymethyl)-benzylalkohol in den Monosilylether der Formel V überführt. 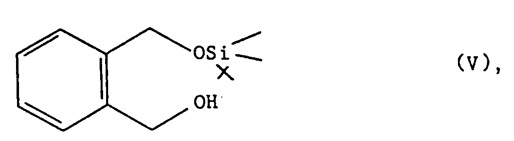
Durch Oxidation z.B. mit Braunstein wird der Aldehyd der Formel VI erhalten, 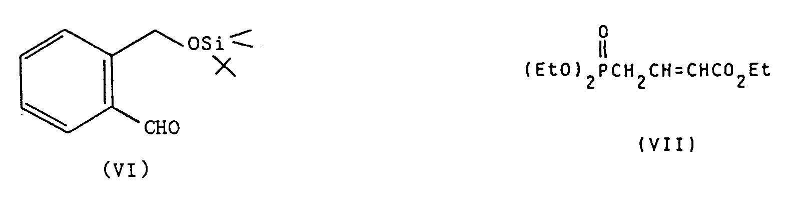
der in einer Wittig-Horner-Olefinierung mit dem Phosphonat der Formel VII und einer Base und gegebenenfalls anschließender Hydrierung in den Ester der Formel VIII überführt wird, wobei A die 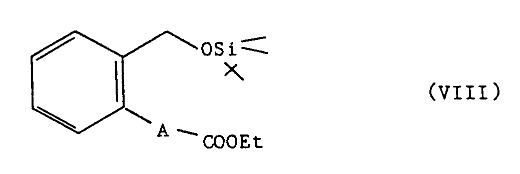
oben angegebene Bedeutung besitzt. Als Basen kommen beispielsweise Kaliumtert.-butylat, Diazabicyclononan oder Natriumhydrid infrage. Reduktion der Estergruppe, beispielsweise mit DIBAH und anschließende Oxidation des erhaltenen primären Alkohols z.B. mit Braunstein oder Collinsreagenz führt zum Aldehyd der Formel IX 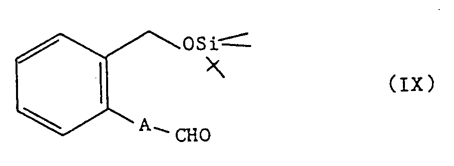
Die metallorganische Umsetzung des Aldehyds der Formel IX mit einem Grignardreagenz der Formel X, worin B, D

X-Mg-B-D-R₄ (X)

und R₄ die oben angegebenen Bedeutungen aufweisen und X Chlor, Brom oder Jod bedeutet, führt nach Schutz der Hydroxygruppe (beispielsweise durch Acylierung) zu den Verbindungen der Formel XI 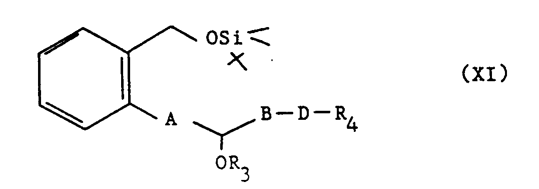
Die Herstellung der für die metallorganische Umsetzung benötigten Verbindung der Formel X erfolgt durch Reaktion des entsprechenden terminalen Halogenids mit Magnesium. Durch Umsetzung des Silylethers XI mit Tetrabutylammoniumfluorid wird der Alkohol der Formel XII erhalten. 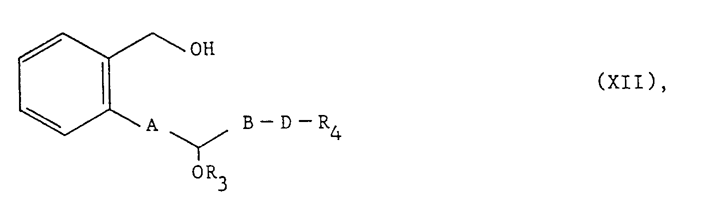
Die Oxidation der primären Alkoholgruppe in XII z. B. mit Braunstein oder Collinsreagenz oder Pyridiniumdichromat führt zum Aldehyd der Formel II.

Die Verbindungen der Formel I wirken antientzündlich und antiallergisch. Daneben besitzen sie antimykotische Eigenschaften. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Verbrennungen, Tinea, Pruritis vulvae et ani. Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigeneten Zusätzen in die gewünschte Applikationsform, wie zum Beispiel: Lösungen, Lotionen, Salben, Cremes oder Pflaster, überführt, In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise o,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten, Thromboxanantagonisten, Leukotrien D₄-antagonisten, Leukotrien E₄-antagonisten, Leukotrien F₄-antagonisten, Phosphodiesterasehemmern oder PAF-Antagonisten verwendet werden.

Die Verbindungen dieser Erfindung können z.B. analog zu dem bekannten antiinflammatorischen und antiallergischen Wirkstoff Dexamethason, einem Glucocorticoid, verabreicht werden. Eine typische systemisch verwendete Dosis für die genannten Indikationen wäre 0,1-50mg/kg/Tag oder bei topischer Verabreichung 1-10 mal am Tag in konventionellen, oben bereits genannten Formulierungen. Eine besondere Dosismenge für eine bestimmte indikation, Patienten und Verbindung kann routinemäßig bestimmt werden, z.B. durch Benutzung der pharmazeutischen Standardvorschriften in Verbindung mit dem in "Models in Dermatology 1987, ed. A.J. Maibach, N.J. Lowe, Karger Basel" beschriebenen Standardprotokoll. Der Dosisbereich für Inhalationsmittel ist 0,01-5 mg/kg/Tag.

Ob eine Verbindung als Antagonist, als Agonist oder als Partialantagonist wirkt, kann in jedem Fall routinemäßig bestimmt werden durch Nutzung eines konventionellen pharmakologischen Protokolls, welches
1. die Fähigkeit einer Verbindung mit LTB₄-Rezeptoren (Methods in Enzymology 163, 1988, page 340-344) in Wechselwirkung zu treten oder
2. die Fähigkeit der Verbindung, die LTB₄ induzierte Chemotaxis weißer Blutzellen in vitro (Methods in Enzyanology 162, 1988, page 38-50) zu erhöhen oder zu hemmen, mißt.

Somit binden alle Verbindungen dieser Erfindung an den LTB₄-rezeptor und weisen in vitro in einem funktionalen Test wie Chemotoxis antagonistische, agonistische oder partialantagonistische Funktionen auf.
Antogonisten sind nützlich bei Hauterkrankungen, die einhergehen mit Entzündungprozessen (gesteigerter Gefäßdurchlässigkeit und Ödem-Bildung. Zellinfiltration), gesteigerter Proliferation der Hautzellen und Juckreiz, wie z.B. bei Ekzemen, Erythemen, Psoriasis, Pruritis, Akne sowie ulcerativer Colitis und asthmatischen Lungenkrankheiten. Agonistische Verbindungen sind potentiell nützlich in der Behandlung von Krankheiten, bei denen die Unterstützung des physiologischen Abwehrmechanismus hilfreich ist, z.B. bei Pilzerkrankungen der Haut.
Partialantagonisten lassen sich entsprechend Terence P. Kenakin: Pharmacologic Analysis of Drug-Receptor Interactions, 1987, Raven Press definieren.

Ein Beispiel für einen Agonisten ist z.B. 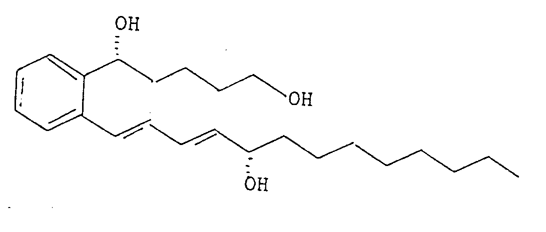
Beispiele für Antagonisten sind z.B.

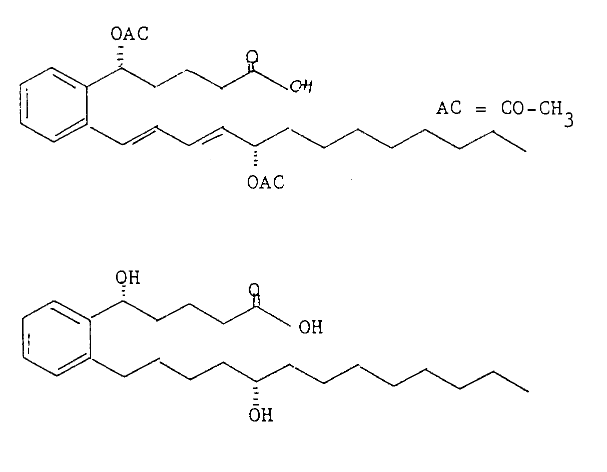

### Beispiel 1

### (1RS)-1-[2-((1E,3E)-(5RS)-5-Acetoxy-1,3-tridecadienyl)-phenyl]-1-acetoxy-5-pentanol

In 960 mg Magnesium tropft man bei 25 °C unter Argon eine Lösung aus 4 g 4-Chlor-1-tetrahydropyranyloxy-butan in 4 ml Tetrahydrofuran und 0,125 Dibrommethan, erwärmt 5 Minuten auf 70 °C, rührt 30 Minuten bei 25 °C und verdünnt mit 12,5 ml Tetrahydrofuran.

Zu 10 ml dieser magnesiumorganischen Lösung tropft man bei -70 °C unter Argon eine Lösung aus 1,5 g 2-((1E,3E)-(5RS)-5-Acetoxy-1,3-tridecadienyl)-benzaldehyd in 15 ml Tetrahydrofuran und rührt 2 Stunden bei -70 °C. Man versetzt mit 50 ml gesättigter Ammoniumchloridlösung, extrahiert dreimal mit Ether, schüttelt die organische Phase mit Sole, trocknet über Magnesiumsulfat und dampft im Vakuum ein. Den Rückstand chromatographiert man an Kieselgel. Mit Hexan-Essigester (6+4) erhält man 2,9 g (1RS)-1-[2-((1E,3E)-(5RS)-5-Acetoxy-1,3-tridecadienyl)-phenyl]-1-hydroxy-5-tetrahydropyranyloxy-pentan als farbloses Öl.
IR (CHCL₃) : 3600, 3450, 2930, 2860, 1727, 1245, 988 cm⁻¹

Zur Acetylierung fügt man zu einer Lösung von 2,7 g des vorstehend beschriebenen Alkohols in 10 ml Pyridin 5 ml Essigsäureanhydrid und rührt 16 Stunden bei 24 °C. Anschließend engt man im Vakuum unter Zusatz von Toluol ein und chromatographiert den Rückstand an Kieselgel. Mit Hexan/Essigester (4+1) erhält man 2,4 g des Acetats als farbloses Öl.
IR: 2930, 2860, 1729, 1245, 989 cm⁻¹

Zur Schutzgruppenabspaltung rührt man 2,3 g des vorstehend hergestellten Acetats mit 50 ml einer Mischung aus Essigsäure/Wasser/Tetrahydrofuran (65+35+10) 20 Stunden bei 25 °C. Anschließend engt man unter Zusatz von Toluol ein und chromatographiert den Rückstand an Kieselgel. Mit Hexan/Essigester (4+6) erhält man 1,1 g der Titelverbindung als farbloses Öl. Als polarere Hebenkomponente eluiert man noch 0,5 g (1RS)-1-[2-((1E,3E)-(5RS)-5-hydroxy-1,3-tridecadienyl)-phenyl]-1-acetoxy-5-pentanol.
IR der Titelverbindung: 3610, 3450, 2930, 2859, 1730, 1245, 988 cm⁻¹
Das Ausgangsmaterial für die obige Titelverbindung wird wie folgt hergestellt:
1a) 5-[2-tert.-Butyl-dimethylsilyloxy-methyl)-phenyl]-(2E,4E)-pentadiensäureethylester
   Zu einer Lösung von 20 g 2-Hydroxymethylbenzylalkohol und 20 g Imidazol in 170 ml Dimethylformamid fügt man bei 0 °C unter Argon 22,16 g tert.-Butyldimethylsilylchlorid und rührt 16 Stunden bei 25 °C. Man verdünnt mit 1,6 l Ether, schüttelt zweimal mit je 100 ml 10 %iger Schwefelsäure, wäscht mit Sole neutral, trocknet über Magnesiumsulfat und dampft im Vakuum ein. Den Rückstand chromatographiert man an Kieselgel. Mit Hexan/ Ether (6+4) erhält man 17 g 2-tert.Butyldimethylsilyloxymethylbenzylalkohol als farbloses Öl.
   IR: 3600, 3450, 2953, 2930, 2860, 835 cm⁻¹
   Eine Lösung aus 16,3 g des vorstehend hergestellten Silylethers in 250 ml Methylenchlorid versetzt man mit 45 g Braunstein und rührt 5 Stunden bei 25 °C. Anschließend wird filtriert, eingedampft und an Kieselgel chromatographiert. Mit Hexan/Ether (6+4) erhält man 15 g 2-tert.-Butyldimethylsilyloxymethylbenzaldehyd als farbloses Öl.
   IR: 2958, 2930, 2859, 1692, 1600, 1572, 836 cm⁻¹
   Zur Wittig-Horner-Olefimierung fügt man bei - 20 °C 6,4 g Kalium-tert.-butylat zu einer Lösung aus 15 g Phosphoncrotonsäuretriethylester und rührt 30 Minuten bei - 20 °C. Anschließend tropft man in diese Lösung eine Lösung aus 15 g 2-tert.-Butyldimethylsilyloxymethylbenzaldehyd und rührt 1 Stunde bei - 20 °C. Man gießt dann auf 200 ml Eiswasser, extrahiert dreimal mit je 200 ml Ether, wäscht die organische Phase mit Wasser neutral, trocknet über Magnesiumsulfat und dampft im Vakuum ein. Den Rückstand chromatographiert man an Kieselgel. Mit Hexan/Ether (6+4) erhält man 15,4 g der Titelverbindung als farbloses Öl.
   IR: 2958, 2930, 2858, 1700, 1627, 1255, 998, 838 cm⁻¹
1b) 1-[2-(tert.-Butyl-dimethylsilyloxy-methyl)-phenyl]-(1E,3E)-pentadien-5-al
   Zu einer Lösung von 17,5 g des nach Beispiel 1a hergestellten Esters in 400 ml Toluol tropft man bei - 70 °C unter Argon 84 ml einer ca. 1,2 molaren Lösung von Diisobutylaluminiumhydrid in Toluol und rührt 30 Minuten bei - 70 °C. Anschließend tropft man 30 ml Isopropanol und dann 40 ml Wasser zu, rührt 2 Stunden bei 22 °C, filtriert, wäscht mit Methylenchlorid und dampft im Vakuum ein. Den Rückstand reinigt man durch Chromatographie an Kieselgel. Mit Hexan/Essigester (4+6) erhält man 11,9 g 1-[2-(tert.-Butyl-dimethylsilyloxy-methyl)-phenyl]-(1E,3E)-pentadien-5-ol als farbloses Öl.
   IR: 3610, 3450, 2958, 2930, 1255, 990, 838 cm⁻¹
   Eine Lösung von 8,5 g des vorstehend hergestellten Alkohols in 250 ml Methylenchlorid versetzt man mit 40 g Braunstein und rührt 4 Stunden bei 25 °C. Anschließend wird filtriert, eingedampft und an Kieselgel chromatographiert. Mit Hexan/Essigester (8+2) eluiert man 7 g der Titelverbindung als farbloses Öl.
   IR: 2958, 2930, 2860, 2741, 1675, 1620, 1600, 985, 838 cm⁻¹
1c) 5-Acetoxy-1-[2-(tert.-Butyl-dimethylsilyloxymethyl)-phenyl]-(1E,3E)-tridecadien
   Zu 1,12 g Magnesium in 5 ml Ether tropft man unter Erwärmen eine Lösung aus 7,96 ml Octylbromid in 12 ml Ether und rührt 30 Minuten bei 25 °C. Zu 12 ml (≙ 22,8 mM) dieser Grignardlösung tropft man bei - 20 °C unter Argon eine Lösung aus 6,2 (≙ 20,5 mM) des nach Beispiel 1b hergestellten Aldehyds in 100 ml Ether und rührt 2 Stunden bei - 20 °C. Man versetzt mit 60 ml gesättigter Ammoniumchloridlösung, extrahiert dreimal mit Ether, schüttelt die organische Phase mit Sole, trocknet über Magnesiumsulfat und dampft im Vakuum ein. Den Rückstand chromatographiert man an Kieselgel. Mit Hexan/Essigester (6+4) eluiert man 6,5 g 5-Hydroxy-1-[2-(tert.-Butyl-dimethylsilyloxymethyl)-phenyl]-(1E,3E)-tridecadien als farbloses Öl.
   IR: 3610, 3450, 2930, 2860, 989, 839 cm⁻¹
   Zur Acetylierung fügt man zu einer Lösung von 6,4 g des vorstehend hergestellten Alkohols in 25 ml Pyridin 12 ml Essigsäureanhydrid und rührt 16 Stunden bei 24 °C. Anschließend engt man im Vakuum unter Zusatz von Toluol ein und chromatographiert den Rückstand an Kieselgel. Mit Hexan/Ether (8+2) eluiert man 6,2 g der Titelverbindung als farbloses Öl.
   IR: 2930, 2858, 1726, 1250, 887, 838 cm⁻¹
1d) 2-((1E,3E)-(5RS)-5-Acetoxy-1,3-tridecadienyl)-benzaldehyd
   Zu einer Lösung von 6,1 g des nach Beispiel 1c hergestellten Acetats in 400 ml Tetrahydrofuran fügt man bei 0 °C 12,3 g Tetrabutylammoniumfluorid, rührt 0,5 Stunden bei 0 °C und 3 Stunden bei 24 °C. Anschließend verdünnt man mit 400 ml Ether, schüttelt dreimal mit je 200 ml Sole, trocknet die organische Phase über Magnesiumsulfat und dampft im Vakuum ein. Den Rückstand chromatographiert man an Kieselgel. Mit Hexan/Essigester (6+4) eluiert man 4,5 g des Benzylalkohols als farbloses Öl.
   IR : 3600, 2925, 2855, 1725, 1255, 988 cm⁻¹
   Zu einer Lösung von 2,3 g des vorstehend hergestellten Benzylalkohols in 60 ml Methylenchlorid fügt man 8 g Braunstein und rührt 4 Stunden bei 24 °C. Anschließend wird filtriert, mit Methylenchlorid gewaschen und im Vakuum eingedampft. Den Rückstand chromatographiert man mit Hexan/Essigester (4+1) an Kieselgel. Dabei erhält man 2,2 g der Titelverbindung als farbloses Öl.
   IR: 2925, 2855, 2740, 1728, 1695, 1598, 1255, 988 cm⁻¹

### Beispiel 2

### (5RS)-5-Acetoxv-5-[2-(1E,3E)-(5RS)-5-acetoxy-1,3-tridecadienyl)-phenyl]-pentansäure

Zu einer Mischung von 3,3 g Collins-Reagenz (Chromsäure-Pyridinkomplex) in 25 ml Methylenchlorid tropft man bei 0 °C unter Rühren eine Lösung von 0,85 g (1RS)-1-[2-((1E,3E)-(5RS)-5-Acetoxy-1,3-tridecadienyl)-phenyl]-1-acetoxy-5-pentanol (hergestellt nach Beispiel 1) in 25 ml Methylenchlorid und rührt 30 Minuten bei 0 °C. Anschließend verdünnt man mit einer Mischung aus Hexan/Ether (2+1), filtriert über Celite und dampft im Vakuum ein. Den Rückstand chromatographiert man an Kieselgel. Mit Hexan/Essigester (4+1) erhält 650 mg (1RS)-1-[2-((1E,3E)-(5RS)-5-Acetoxy-1,3-tridecadienyl)-phenyl]-1-acetoxy-5-pentanal als farbloses Öl.
IR: 2930, 2860, 2730, 1727, 1245, 988 cm⁻¹
Zu einer Lösung von 600 mg des vorstehend hergestellten Aldehyds in 20 ml Aceton tropft man unter Rühren bei - 20 °C 1 ml Jones-Reagenz (J. Chem. Soc. 1953, 2555) und rührt 10 Minuten bei - 20 °C. Anschließend fügt man 5 ml Isopropanol zu, rührt 10 Minuten, verdünnt mit 50 ml Ether, filtriert, wäscht mit Ether, schüttelt die Etherphase zweimal mit Sole, trocknet über Magnesiumsulfat und dampft im Vakuum ein. Den Rückstand chromatographiert man an Kieselgel. Mit Hexan/Essigester (6+4) erhält man 545 mg der Titelverbindung als farbloses Öl. IR: 3520 (breit), 1728, 1245, 988 cm⁻¹

### Beispiel 3

### (1RS)-1-[2-((1E,3E)-(5RS)-5-Hydroxy-1,3-tridecadienyl)-phenyl]-1,5-pentandiol

Zu einer Lösung von 190 mg (1RS)-1-[2-((1E,3E)-(5RS)-5-hydroxy-1,3-tridecadienyl)-phenyl]-1-acetoxy-5-pentanol (hergestellt in Beispiel 1) in 4,6 ml Methanol fügt man bei 24 °C 4,6 ml einer 0,5 normalen Natronlauge und rührt 30 Minuten bei 24 °C unter Argon. Anschließend verdünnt man mit 10 ml Wasser, säuert mit 10 %iger Citronensäurelösung auf pH 5-6 an, extratriert viermal mit je 30 ml Essigester, schüttelt die organische Phase mit Sole, trocknet über Magnesiumsulfat und dampft im Vakuum ein. Den Rückstand chromatographiert man an Kieselgel. Mit Essigester/Hexan (4+1) erhält man 113 mg der Titelverbindung als farbloses Öl.
IR: 3610, 3450, 2930, 2860, 988 cm-1

### Beispiel 4

### (5RS)-5-Acetoxy-5-[2-(5RS)-5-acetoxy-tridecanyl)-phenyl]-pentansäure

Eine Lösung von 150 mg (5RS)-5-Acetoxy-5-[2-(1E,3E)-(5RS)-5-acetoxy-1,3-tridecadienyl)-phenyl]-pentansäure in 5 ml Essigester versetzt man mit 15 mg Palladium 10 %ig auf Kohle und schüttelt die Suspension 3 Stunden bei 24 °C unter einer Wasserstoffatmosphäre. Anschließend wird filtriert und im Vakuum eingedampft. Den Rückstand chromatographiert man an Kieselgel. Dabei erhält man neben unpolaren Nebenprodukten 91 mg der Titelverbindung als farbloses Öl.
IR: 3510 (breit), 2930, 2860, 1727, 1250 cm⁻¹

### Beispiel 5

### (5RS)-5-Hydroxy-5-[2-((5RS)-5-hydroxy-tridecanyl)-phenyl]-pentansäure

Zu einer Lösung von 13 mg des nach Beispiel 5 hergestellten Hydrierungsproduktes in 0,27 ml Methanol fügt man bei 24 °C 0.27 ml einer 0,5 normalen Natronlauge und rührt 16 Stunden bei 24 °C unter Argon. Anschließend verdünnt man mit 2 ml Wasser, säuert bei 0 °C mit 10 %iger Citronensäurelösung auf pH 4 an, extrahiert dreimal mit Methylenchlorid, schüttelt den organischen Extrakt mit Sole, trocknet über Magnesiumsulfat und dampft im Vakuum ein. Den Rückstand chromatographiert man an Kieselgel. Mit Essigester/Hexan (4+1) eluiert man 9 mg der Titelverbindung als farbloses Öl.
IR: 3500 (breit), 2928, 2559, 1710 cm⁻¹

### Beispiel 6

### (5RS)-5-Acetoxy-5-[2-(1E,3E)-(5RS)-5-acetoxy-1,3-tridecadienyl)-phenyl]-pentansäuremethylester

Zu einer Lösung von 120 mg der nach Beispiel 2 hergestellten Säure in 6 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (9+1) erhält man 103 mg der Titelverbindung als farbloses Öl.
IR: 2930, 2858, 1735, 1245, 988 cm⁻¹

### Beispiel 7

### (5RS)-5-Acetoxy-5-[2-(5RS)-5-acetoxy-tridecanyl)-phenyl]-pentansäuremethylester

Zu einer Lösung von 43 mg der nach Beispiel 5 hergestellten Säure in 3 ml Methylenchlorid tropft man bei 0 °C eine ätherische Diazomethanlösung bis zur bleibenden Gelbfärbung und rührt 15 Minuten bei 0 °C. Anschließenden dampft man im Vakuum ein und chromatographiert den Rückstand an Kieselgel. Mit Hexan/Essigester (9+1) erhält man 33 mg der Titelverbindung als farbloses Öl.
IR: 2929, 2858, 1736, 1250 cm⁻¹

### Beispiel 8

### (5RS)-5-Hydroxy-5-[2-((5RS)-5-hydroxy-tridecanyl)-phenyl]-pentansäuremethylester

Zu einer Lösung von 19 mg der nach Beispiel 6 hergestellten Säure in 2 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (3+2) erhält man 16 mg der Titelverbindung als farbloses Öl.
IR: 3590, 2928, 2560, 1735 cm⁻¹

### Beispiel 9

### (5RS)-5-Benzoyloxy-5-[2-((1E,3E)-(5RS)-5-Benzoyloxy-1,3-tridecadienyl)-phenyl]-pentansäuremethylester

Zu einer Lösung von 190 mg des nach Beispiel 8 hergestellten Methylesters in 3 ml Pyridin fügt man bei 24 °C 170 mg Benzoylchlorid und rührt 16 Stunden bei 24 °C. Anschließend fügt man 0,3 ml Wasser zu, rührt 2 Stunden, verdünnt mit Ather und schüttelt nacheinander mit Wasser 5 %iger Schwefelsäure, Wasser. 4 %ige Natriumhydrogencarbonatlösung und Wasser. Man trocknet über Magnesiumsulfat, dampft im Vakuum und chromatographiert den Rückstand an Kieselgel. Mit Hexan/Essigester (9+1) erhält man 185 mg der Titelverbindung als farbloses Öl.
IR: 2930, 2860, 1733, 1250, 988 cm⁻¹

## Patentansprüche

1. Leukotrien-B₄-Derivate der Formel I, 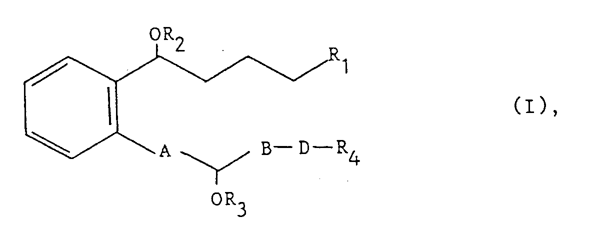 worin
R₁ den Rest CH₂OH, den Rest COOR₅ mit R₅ in der Bedeutung eines Wasserstoffatoms, eines Alkylrestes mit 1-10 C-Atomen, eines Cycloalkylrestes mit 3-10 C-Atomen, eines gegebenenfalls durch 1-3 Chlor, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrestes mit 6-10 C-Atomen, eines -CH₂-CO-Aryl-Restes mit 6-10 C-Atomen für Aryl oder eines 5-6-gliedrigen heterocyclischen Restes mit wenigstens 1 Heteroatom oder den Rest CONHR₆ mit R₆ in der Bedeutung eines Alkanoyl- oder Alkansulfonylrestes mit 1-10 C-Atomen oder des Restes R₅,
A eine trans,trans-CH=CH-CH=CH- oder Tetramethylengruppe,
B eine geradkettige oder verzweigtkettige gesättigte oder ungesättigte Alkylengruppe mit bis zu 10 C-Atomen, die gegebenenfalls durch Fluor substituiert sein kann, oder die Gruppe 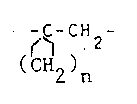 mit n = 1, 2 oder 3,
D eine Direktverbindung, Sauerstoff, Schwefel, eine -C≡C-Gruppe oder eine -CH=CR₇-Gruppe mit R₇ als Wasserstoff, C₁-C₅-Alkyl, Chlor oder Brom,
B und D gemeinsam eine direkte Bindung,
R₂ und R₃ gleich oder verschieden sein können und ein Wasserstoffatom oder einen Säurerest einer organischen Säure mit 1-15 C-Atomen und
R₄ ein Wasserstoffatom, einen Alkylrest mit 1-10 C-Atomen, einen Cycloalkylrest mit 3-10 C-Atomen, einen gegebenenfalls durch 1-2 Chlor, Brom, Phenyl, Alkyl mit 1-4 C-Atomen, Chlormethyl, Fluormethyl, Trifluormethyl, Ccrboxy, C₁-C₄-Alkoxy oder Hydroxy substituierten Arylrest mit 6-10 C-Atomen oder einen 5-6-gliedrigen heterocyclischen Rest mit wenigstens 1 Heteroatom bedeuten
und, falls R₅ die Bedeutung eines Wasserstoffatoms hat, deren Salze mit physiologisch verträglichen Basen und deren Cyclodextrinclathrate,
mit der Maßgabe, daß R₂ und R₃ nicht jeweils Wasserstoff bedeuten, falls A einen trans,trans-CH=CH-CH=CH- und R₁ den Rest -COOR₅ und -CONHR₆ darstellen.

2. Verfahren zur Herstellung von Leukotrien-B₄-Derivaten der Formel I, dadurch gekennzeichnet, daß man einen Benzaldehyd der Formel II. 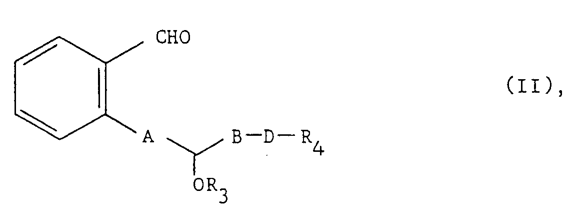 worin A, B, D, R₃ und R₄ die oben angegebenen Bedeutungen haben, gegebenenfalls nach Schutz freier Hydroxygruppen mit einer magnesiumorganischen Verbindung der Formel III.
X-Mg-CH₂-CH₂-CH₂-CH₂-O-R₈ (III).
worin X Chlor, Brom oder Jod und R₈ einen leicht abspaltbaren Ätherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R₁=COOR₅) verseift und/oder reduziert und/oder eine Carboxylgruppe (R₅=H) Verestert und/oder eine freie Carboxylgruppe (R₅=H) in ein Amid (R₁=CONHR₆) überführt oder eine Carboxygruppe mit einer physiologisch verträglichen Base in ein Salz überführt.

3. Arzneimittel bestehend aus einer oder mehreren Verbindungen gemäß Anspruch 1 und üblichen Hilfs- und Trägerstoffen.

## Claims

1. Leukotriene-B₄ derivatives of the formula I 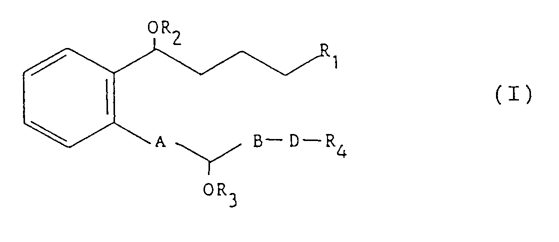 wherein
R₁ represents the radical CH₂OH; the radical COOR₅ in which R₅ represents a hydrogen atom, an alkyl radical having from 1 to 10 carbon atoms, a cycloalkyl radical having from 3 to 10 carbon atoms, an aryl radical having from 6 to 10 carbon atoms that is optionally mono- to tri-substituted by chlorine, bromine, phenyl, alkyl having from 1 to 4 carbon atoms, chloromethyl, fluoromethyl, trifluoromethyl, carboxy, C₁-C₄-alkoxy or by hydroxy, a -CH₂-CO-aryl radical having from 6 to 10 carbon atoms for aryl, or a 5- or 6-membered heterocyclic radical having at least one hetero atom; or R₁ represents the radical CONHR₆, in which R₆ represents an alkanoyl or alkanesulphonyl radical having from 1 to 10 carbon atoms or the radical R₅,
A represents a trans,trans-CH=CH-CH=CH- or tetramethylene group,
B represents a straight-chain or branched-chain saturated or unsaturated alkylene group having up to 10 carbon atoms that may optionally be substituted by fluorine, or represents the group 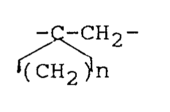 in which n = 1, 2 or 3,
D represents a direct bond, oxygen, sulphur, a -C≡C-group or a -CH=CR₇- group in which R₇ represents hydrogen, C₁-C₅-alkyl, chlorine or bromine,
B and D together represent a direct bond,
R₂ and R₃ may be identical or different and represent a hydrogen atom or an acid radical of an organic acid having from 1 to 15 carbon atoms and
R₄ represents a hydrogen atom, an alkyl radical having from 1 to 10 carbon atoms, a cycloalkyl radical having from 3 to 10 carbon atoms, an aryl radical having from 6 to 10 carbon atoms that is optionally mono- or di-substituted by chlorine, bromine, phenyl, alkyl having from 1 to 4 carbon atoms, chloromethyl, fluoromethyl, trifluoromethyl, carboxy, C₁-C₄-alkoxy or by hydroxy, or represents a 5- or 6-membered heterocyclic radical having at least one hetero atom,
and, if R₅ represents a hydrogen atom, the salts thereof with physiologically tolerable bases and the cyclodextrin clathrates thereof,
with the proviso that R₂ and R₃ do not each represent hydrogen when A represents trans,trans-CH=CH-CH=CH- and R₁ represents the radical -COOR₅ or -CONHR₆.

2. Process for the preparation of leukotriene-B₄ derivatives of the formula I, characterised in that a bensaldehyde of the formula II, 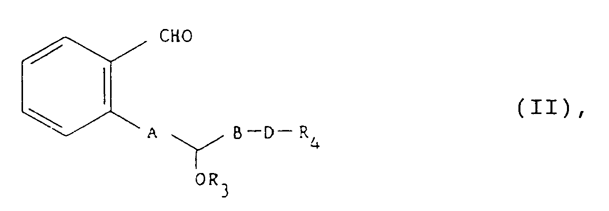 wherein A, B, D, R₃ and R₄ are as defined above, is reacted, optionally after protecting free hydroxy groups, with an organomagnesium compound of the formula III
X-Mg-CH₂-CH₂-CH₂-CH₂-O-R₈ (III),
wherein X is chlorine, bromine or iodine and R₈ is a readily removable ether radical, and then, optionally, in any sequence, isomers are separated, protected hydroxy groups are freed and/or a free hydroxy group is esterified and/or the 1-hydroxy group is oxidised to the carboxylic acid and/or double bonds are hydrogenated and/or an esterified carboxy group (R₁=COOR₅) is hydrolysed and/or reduced and/or a carboxy group (R₅=H) is esterified and/or a free carboxy group (R₅=H) is converted into an amide (R₁=CONHR₆) or a carboxy group is converted with a physiologically tolerable base into a salt.

3. Medicament comprising one or more compounds according to claim 1 and customary excipients and carriers.

## Revendications

1. Dérivés du leucotriène-B4 de formule I : 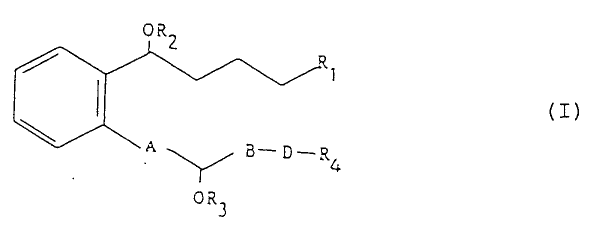 (dans laquelle
R₁ représente le radical CH₂OH, un radical COOR₅, R₅ désignant un atome d'hydrogène, un alkyle en C₁-C₁₀, un cycloalkyle en C₃-C₁₀, un aryle en C₆-C₁₀ avec éventuellement comme substituants de 1 à 3 atomes de chlore ou de brome ou groupes phényle, alkyles en C₁-C₄, chlorométhyle, fluorométhyle, trifluorométhyle, carboxy, alcoxy en C₁-C₄ ou hydroxy, un radical -CH₂-CO-aryle dont l'aryle a de 6 à 10 atomes de carbone ou un radical hétérocyclique à 5 ou 6 chaînons et au moins un hétéroatome, ou bien un radical CONHR₆, R₆ étant un groupe alcanoyle ou alcane-sulfonyle en C₁-C₁₀ ou le radical R₅ ci-dessus,
A représente un groupe trans,trans-CH=CH-CH=CH- ou un groupe tétraméthylène,
B un alkylène linéaire ou ramifié, saturé ou insaturé, pouvant avoir jusqu'à 10 atomes de carbone et pouvant éventuellement porter du fluor, ou bien le groupe -C-CH₂- dans lequel n est 1, 2 ou 3,
(CH₂)ₙ
D désigne une liaison directe ou bien l'oxygène ou le soufre ou un groupe -C≡C- ou un -CH=CR₇-, R₇ désignant l'hydrogène, un alkyle en C₁-C₅ ou le chlore ou le brome, ou bien
B et D représentent ensemble une liaison directe,
R₂ et R₃, qui peuvent être identiques ou différents l'un, de l'autre, sont chacun un atome d'hydrogène ou le radical d'un acide organique en C₁-C₁₅, et
R₄ représente un atome d'hydrogène, un alkyle en C₁-C₁₀ éventuellement chloré ou bromé, un cycloalkyle en C₃-C₁₀, un aryle en C₆-C₁₀ avec éventuellement comme substituants un à deux atomes de chlore ou de brome ou groupes phényle, alkyles en C₁-C₄, chlorométhyle, fluorométhyle, trifluorométhyle, carboxy, alcoxy en C₁-C₄ ou hydroxy, ou encore un radical hétérocyclique à 5 ou 6 chaînons et avec au moins un hétéroatome, avec la condition que R₂ et R₃ ne soient pas tous deux l'hydrogène si A est un groupe trans,trans-CH=CH-CH=CH- et R₁ le radical -COOR₅ ou -CONHR₆), ainsi que, si R₅ est un atome d'hydrogène, les sels de ces dérivés avec des bases physiologiquement comptabibles et tolérées et leurs clathrates de cyclodextrines.

2. Procédé de préparation des dérivés de leucotriène-B4 de formule I, procédé caractérisé en ce que l'on fait réagir un benzaldéhyde de formule II : 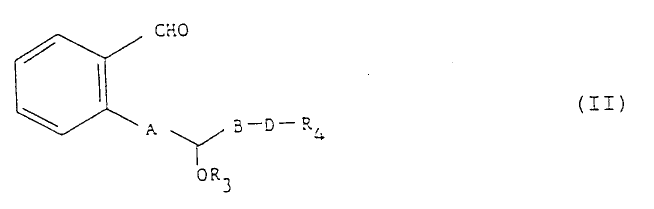 les divers symboles ayant les significations précédemment données, le cas échéant après avoir protégé des hydroxyles libres, avec un composé organomagnésien de formule III :
X-Mg-CH₂-CH₂-CH₂-CH₂-O-R₈ (III)
dans laquelle X désignant le chlore, le brome ou l'iode et R₈ un radical d'éther facilement éliminable, puis éventuellement, et dans un ordre quelconque, on sépare les isomères, on libère des hydroxyles protégés, et/ou on estérifie un hydroxyle libre et/ou on oxyde le groupe 1-hydroxy en acide carboxylique et/ou on hydrogène des doubles liaisons et/ou on saponifie un groupe carboxylique estérifié (R₁ = COOR₅) et/ou on le réduit et/ou on estérifie un groupe carboxylique (R₅ = H) et/ou on transforme un groupe carboxylique libre (R₅ = H) en un amide (R₁ = CONHR₆), ou encore on salifie un groupe carboxylique avec une base physiologiquement compatible.

3. Médicaments comprenant un ou plusieurs composés de la revendication 1 avec des adjuvants et véhicules courants.
